(19) Europäisches Patentamt
European Patent Office
Office européen des brevets

(11) EP 1 330 309 B1

(12) **EUROPÄISCHE PATENTSCHRIFT**

(45) Veröffentlichungstag und Bekanntmachung des Hinweises auf die Patenterteilung:
**05.04.2006 Patentblatt 2006/14**

(21) Anmeldenummer: **01978386.9**

(22) Anmeldetag: **25.09.2001**

(51) Int Cl.:
***B01J 31/16*** *(2006.01)* ***C07C 51/14*** *(2006.01)*
***C07C 67/38*** *(2006.01)*

(86) Internationale Anmeldenummer:
**PCT/EP2001/011052**

(87) Internationale Veröffentlichungsnummer:
**WO 2002/026382 (04.04.2002 Gazette 2002/12)**

(54) **KATALYSATORSYSTEM UND VERFAHREN ZUR CARBONYLIERUNG**

CATALYST SYSTEM AND METHOD FOR CARBONYLATION

SYSTEME CATALYSEUR ET PROCEDE DE CARBONYLATION

(84) Benannte Vertragsstaaten:
**AT BE CH CY DE DK ES FI FR GB GR IE IT LI LU MC NL PT SE TR**

(30) Priorität: **29.09.2000 DE 10048874**

(43) Veröffentlichungstag der Anmeldung:
**30.07.2003 Patentblatt 2003/31**

(73) Patentinhaber: **BASF Aktiengesellschaft**
**67056 Ludwigshafen (DE)**

(72) Erfinder:
- **SCHÄFER, Martin**
**67269 Grünstadt (DE)**
- **SLANY, Michael**
**67281 Kirchheim (DE)**
- **ZELLER, Edgar**
**68165 Mannheim (DE)**
- **RÖPER, Michael**
**67157 Wachenheim (DE)**
- **SCHULZ, Michael**
**67550 Worms (DE)**
- **GRIMM, Günther**
**67069 Ludwigshafen (DE)**

(56) Entgegenhaltungen:

| | |
|---|---|
| **EP-A- 0 008 407** | **EP-A- 0 441 446** |
| **WO-A-01/10551** | **US-A- 4 127 506** |

Bemerkungen:
Die Akte enthält technische Angaben, die nach dem Eingang der Anmeldung eingereicht wurden und die nicht in dieser Patentschrift enthalten sind.

EP 1 330 309 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein Katalysatorsystem zur Carbonylierung von olefinisch oder acetylenisch ungesättigten Verbindungen mit Kohlenmonoxid und einer nukleophilen Verbindung, enthaltend

(a) Palladium;
(b) ein Phosphin; und
(c) ein im Reaktionsgemisch homogen gelöstes oder zumindest kolloidal verteiltes, acyliertes Polyethylenimin, welches Einheiten der allgemeinen Formel (I) oder verzweigter Isomere davon

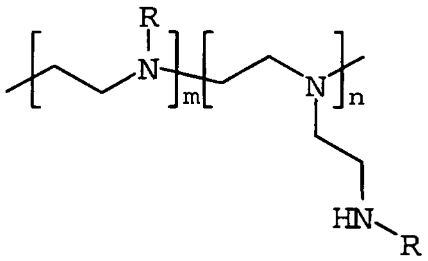

(I),

in der die Summe aus m+n mindestens 10 beträgt und R jeweils unabhängig voneinander Wasserstoff, Alkyl-, Cycloalkyl-, Aryl-, Aralkyl-, Acyl-Reste mit bis zu 30 Kohlenstoffatomen oder Hydroxyalkyl(poly)oxyalkylen-Reste mit bis zu 500 Alkylenoxy-Einheiten bedeuten, aufweist, wobei das Polyethylenimin (I) die folgenden Strukturelemente (II) oder verzweigte Isomere davon

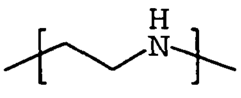

,

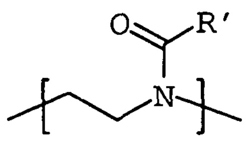

,

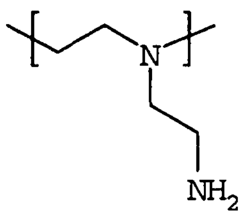

und

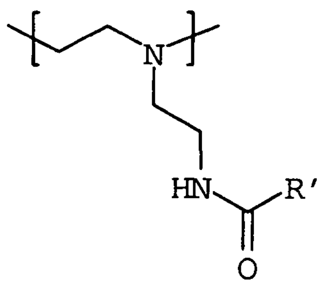

(II),

in denen R' unabhängig voneinander Alkyl mit 1 bis 29 Kohlenstoffatomen bedeutet, aufweist, und dessen Amidierungsgrad 1 bis nahe 100 %, bezogen auf die amidierbaren Aminogruppen beträgt;

sowie ein Verfahren zur Carbonylierung in Gegenwart eines solchen Katalysatorsystems.

Die Carbonylierung von olefinisch ungesättigten Verbindungen mit Kohlenmonoxid und einer nukleophilen Verbindung, wie Wasser, eines Alkohols oder einer Carbonsäure, zur Herstellung von Carbonsäuren, Carbonsäureestern und Carbonsäureanhydriden in Gegenwart eines Palladium und Phosphin enthaltenden Katalysatorsystems ist seit langem bekannt und beispielsweise in EP-A 0 055 875 beschrieben. Nachteilig bei diesem Verfahren ist der erforderliche hohe Druck von 25 bis 100 bar (2,5 bis 10 MPa) und die niedrige Reaktionsgeschwindigkeit.

EP-A 0 106 379 lehrt eine deutliche Steigerung der Reaktionsgeschwindigkeit durch Zusatz einer starken Protonensäure, wie etwa einer Sulfonsäure. Die Zugabe einer Protonensäure als Promotor hat jedoch den Nachteil, daß diese mit dem Reaktionsprodukt ausgetragen wird und vor allem auch vom Reaktionsprodukt abgetrennt werden muß.

In F. Bertoux, J. Mol. Catal. A 143 (1999) Seiten 23 bis 30 ist die Carbonylierung von Propen zu Buttersäure in Gegenwart eines katalytischen System, welches durch Zusammenfügen von Palladiumchlorid, Tris(m-sulfonatophenyl)phosphin Trinatriumsalz, Chlorwasserstoff und Polyvinylalkohol in Wasser und anschließender Aktivierung unter Reaktionsbedingungen hergestellt wurde. Die Carbonylierung wurde in einem Zweiphasensystem bestehend aus einer wässrigen Phase und einer organischen, Toluol haltigen Phase bei einem CO-Druck von 40 atm (etwa 4 MPa) durchgeführt. Der Polyvinylalkohol wirkt als Lösungsvermittler für das Katalysatorsystem in beiden Phasen. Von Nachteil sind die Gegenwart von Chlorid, der erforderliche hohe Druck von etwa 40 atm und die Durchführung in einem Zweiphasensystem.

EP-A 0 274 795 offenbart die Carbonylierung von Olefinen mit Kohlenmonoxid und Wasser, Alkoholen oder Carbonsäuren in Gegenwart eines Palladium-Katalysators, eines organischen Phosphins, einer Säure mit einem $pK_a$-Wert kleiner 2 und eines Stabilisators. Als Stabilisatoren sind niedermolekulare Carbonsäureamide, wie N-Methyl-pyrrolidon und N,N-Diphenylacetamid, genannt.

In WO 94/18154 ist die Carbonylierung von olefinisch ungesättigten Verbindungen mit Kohlenmonoxid und eines Alkohols oder Amins als Coreaktand in Gegenwart eines Katalysatorsystems, welches eine Palladium-Verbindung, einen bidentaten Phosphinliganden und eine basische Verbindung umfasst, beschrieben. Als basische Verbindungen sind die Stickstoff haltigen, niedermolekularen Verbindungen aus der Reihe der Trialkylamine und der Stickstoffheterocyclen, wie etwa Pyridin-Derivate, genannt.

Da die oben beschriebenen niedermolekularen Verbindungen leicht flüchtig sind, werden diese in der Regel mit dem Reaktionsprodukt aus dem Reaktionssystem ausgetragen. Die Reinisolierung des Carbonylierungsprodukts gestaltet sich daher aufwendig.

Das US-Patent 4,127,506 beschreibt die Herstellung eines im Carbonylierungs-Reaktionsmedium unlöslichen, polymergeträgerten Übergangsmetall-Katalysators für Hydroformylierungsreaktionen durch photokatalysierte Umsetzung einer Übergangsmetall-Ausgangsverbindung und eines festen, polymeren Trägers, welcher Amingruppen innerhalb der Polymerkette oder an die Polymerkette gebunden enthält, unter Bestrahlung mit UV-Licht. Nach der Lehre des US-Patents ist es wesentlich, daß die Polymere im Carbonylierungs-Reaktionsmedium unlöslich sind und eine poröse Struktur aufweisen.

EP-A 0 441 446 beschreibt die Carbonylierung von acetylenisch oder olefinisch ungesättigten Verbindungen mit Kohlenmonoxid und Wasser, Alkoholen oder Carbonsäuren in Gegenwart eines Gruppe VIII-Metalls, eines Phosphins, einer Protonensäure und eines tertiären Amins. Als tertiäre Amine sind die heterocyclischen tertiären Amine (wie Pyridine), die aliphatischen tertiären Amine (wie Dialkylamine), die tertiären Aniline (wie N,N-Dialkylaniline) und vernetztes Polyvinylpyridin offenbart. Nachteilig bei diesem Verfahren ist der erforderliche hohe Druck von insgesamt 50 bar (5 MPa).

EP-A-0008407 beschreibt lösliche, polymere Hydrierkatalysatoren umfassend ein Metall oder Metallionen der 8. Nebengruppe des Periodensystems, ein synthetisches oder halbsynthetisches lösliches Polymer und gegebenenfalls einen niedermolekularen Liganden.

Es bestand daher die Aufgabe, ein Katalysatorsystem zur Carbonylierung von olefinisch oder acetylenisch ungesättigten Verbindungen mit Kohlenmonoxid und einer nukleophilen Verbindung zu finden, welches die oben beschriebenen Nachteile nicht mehr besitzt, bereits unter milden Reaktionsbedingungen eine hohe katalytische Aktivität aufweist, eine hohe Stabilität bezüglich einer Abscheidung von Palladium und Palladiumverbindungen besitzt und die Herstellung von Carbonsäuren und Carbonsäurederivaten in hoher Ausbeute ermöglicht. Des weiteren bestand die Aufgabe, ein Verfahren zur Carbonylierung unter Einsatz eines derartigen Katalysatorsystems zu finden.

Demgemäß wurde ein Katalysatorsystem zur Carbonylierung von olefinisch oder acetylenisch ungesättigten Verbindungen mit Kohlenmonoxid und einer nukleophilen Verbindung gefunden, enthaltend

(a) Palladium;
(b) ein Phosphin; und
(c) ein im Reaktionsgemisch homogen gelöstes oder zumindest kolloidal verteiltes, acyliertes Polyethylenimin, welches Einheiten der allgemeinen Formel (I) oder verzweigter Isomere davon

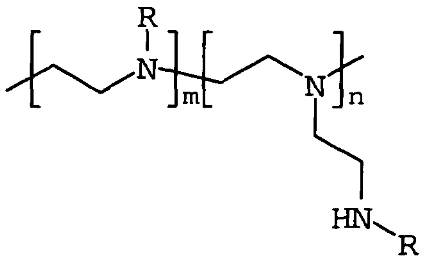

(I),

in der die Summe aus m+n mindestens 10 beträgt und R jeweils unabhängig voneinander Wasserstoff, Alkyl-, Cycloalkyl-, Aryl-, Aralkyl-, Acyl-Reste mit bis zu 30 Kohlenstoffatomen oder Hydroxyalkyl(poly)oxyalkylen-Reste mit bis zu 500 Alkylenoxy-Einheiten bedeuten, aufweist, wobei das Polyethylenimin (I) die folgenden Strukturelemente (II) oder verzweigte Isomere davon

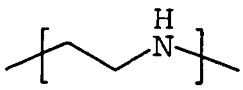

,

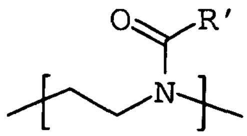

,

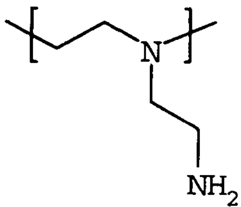

und

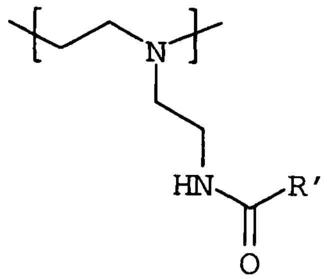

(II),

in denen R' unabhängig voneinander Alkyl mit 1 bis 29 Kohlenstoffatomen bedeutet, aufweist, und dessen Amidierungsgrad 1 bis nahe 100 %, bezogen auf die amidierbaren Aminogruppen beträgt.

Es wurde überraschend gefunden, daß im Reaktionsgemisch homogen gelöste oder zumindest kolloidal verteilte Polyethylenimine den Katalysatorkomplex, welcher Palladium und Phosphin enthält, hervorragend stabilisieren und somit eine Abscheidung von Palladium und von Palladiumverbindungen inhibieren. Zudem verbessern die genannten Polymere die katalytischen Eigenschaften signifikant.

Beim erfindungsgemäßen Katalysatorsystem werden im Reaktionsgemisch homogen gelöste oder zumindest kolloidal verteilte Polyethylenimine eingesetzt, welche vorzugsweise mehr als 10 Stickstoffatome enthalten und eine Molmasse von mehr als 1000 g/mol, vorzugsweise mehr als 10000 bis zu $10^6$ g/mol aufweisen.

Die genannten Polyethylenimine können in einem Molekül unterschiedliche stickstoffhaltige Monomere und gegebenenfalls stickstofffreie Monomere enthalten. Die Stickstoffatome können sich in der Hauptkette befinden oder in Seitengruppen vorliegen.

Die Polyethylenimine sind zur Einstellung einer geeigneten Polarität derivatisiert, zum Beispiel an einem Teil der Aminogruppen oder allen Aminogruppen durch Substituenten, wie beispielsweise Alkyl-, Aryl-, Acyl- oder Polyoxyalkylengruppen. Die Einführung von Substituenten kann durch Derivatisierung mit geeigneten Derivatisierungsreagenzien, wie Carbonsäuren, Carbonsäurederivaten, Alkylierungsmitteln oder Alkenoxiden, durch Phosphonomethylierung, Strekker-Synthese usw. erfolgen. Die Derivatisierung kann an Stickstoffatomen oder an anderen Positionen des Polymeren erfolgen. Die Einführung funktioneller Gruppen kann durch polymeranaloge Umsetzung des Polyethylenimins oder auf der Stufe der zugrundeliegenden Monomeren oder durch Mitverwendung geeigneter copolymerisierbarer stickstofffreier Monomere erfolgen.

Bevorzugt beträgt im Polyethylenimin (I) oder dessen verzweigten Isomeren das Verhältnis aus m/(m+n) 0,01 bis 1.

Die bevorzugt eingesetzten Polyethylenimine (I) enthalten im Allgemeinen primäre ($-NH_2$), sekundäre (>NH)

und tertiäre (>N-) Aminogruppen. Das Verhältnis primärer : sekundärer : tertiärer Aminogruppen beträgt in der Regel 1 : 0,1 bis 2 : 0,1 bis 2 und bevorzugt 1 : 0,8 bis 1,3 : 0,6 bis 1,1.

Als bevorzugte Alkyl-, Cycloalkyl-, Aryl-, Aralkyl- und Acyl-Reste seien die unverzweigten oder verzweigten, gegebenenfalls substituierten $C_1$- bis $C_{20}$-Alkyl-, $C_5$- bis $C_{10}$-Cycloalkyl-, $C_6$- bis $C_{10}$-Aryl-, $C_7$- bis $C_{10}$-Aralkyl- und $C_2$- bis $C_{21}$-Acyl-Reste, wie beispielsweise Methyl, Ethyl, 1-Propyl, 2-Propyl (sek. Propyl), 1-Butyl, 2-Butyl (sek. Butyl), 2-Methyl-1-propyl (iso-Butyl), 2-Methyl-2-propyl (tert.-Butyl), 1-Pentyl, 2-Pentyl, 3-Pentyl, 3-Methyl-2-butyl, 2-Methyl-2-butyl, 1-Hexyl, 1-Heptyl, 1-Octyl, 2-Ethyl-1-hexyl, 1-Nonyl, 1-Decy, 1-Dodecyl, 1-Tetradecyl, 1-Hexadecyl, 1-Octadecyl, 1-Icosyl, Cyclopentyl, Cyclohexyl, Cyclooctyl, Phenyl, 2-Methylphenyl (2-Tolyl), 3-Methylphenyl (3-Tolyl), 4-Methylphenyl (4-Tolyl), 2,4-Dimethylphenyl, 2,6-Dimethylphenyl, 2,4,6-Trimethylphenyl, 1-Naphthyl, 2-Naphthyl und Phenylmethyl (Benzyl) genannt.

Als bevorzugte Alkylenoxy-Einheiten seien die $C_2$- bis $C_6$-Alkylenoxy-Einheiten, wie beispielsweise Ethylenoxy, Propylenoxy, Butylenoxy, Pentylenoxy und Hexylenoxy genannt.

Besonders bevorzugt werden Polyethylenimine (I) oder verzweigte Isomere davon verwendet, bei denen die Reste R jeweils unabhängig voneinander Wasserstoff sowie gleiche oder verschiedene Acylreste mit bis zu 30 Kohlenstoffatomen bedeuten.

Als bevorzugte Alkyl-Reste für R' in den Strukturelementen (II) oder deren verzweigten Isomeren davon seien die unverzweigten oder verzweigten, gegebenenfalls substituierten $C_1$- bis $C_{21}$-Alkyl-Reste, wie beispielsweise Methyl, Ethyl, 1-Propyl, 2-Propyl (sek. Propyl), 1-Butyl, 2-Butyl (sek. Butyl), 2-Methyl-1-propyl (iso-Butyl), 2-Methyl-2-propyl (tert.-Butyl), 1-Pentyl, 2-Pentyl, 3-Pentyl, 1-Hexyl, 1-Heptyl, 2-Heptyl, 3-Heptyl, 1-Octyl, 2,4,4-Trimethylpentyl, 1-Nonyl, 2-Methyl-2-octyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl, 1-Nonadecyl, 1-Icosyl und 1-Henicosyl genannt.

Weitere, beim erfindungsgemäßen Katalysatorsystem besonders bevorzugt eingesetzte Polyethylenimine (I) sind Polyethylenimine, welche als charakteristische Strukturelemente Einheiten der Formel (III) oder verzweigter Isomere davon

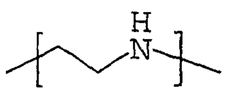

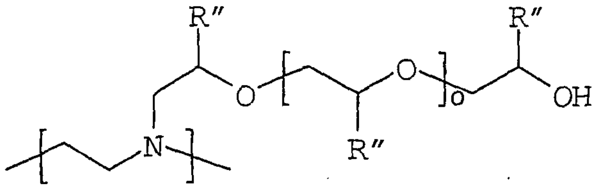
,

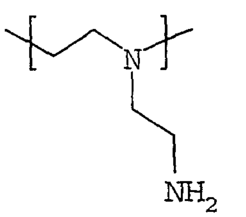

und

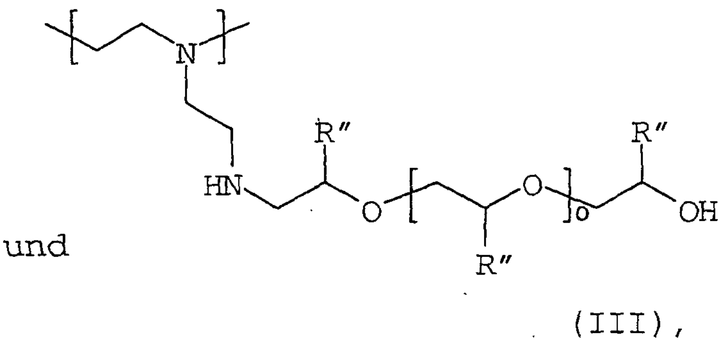

(III),

in denen R" Wasserstoff oder $C_1$- bis $C_4$-Alkyl bedeutet, und o einen Wert von 0 und 500 betragen kann, aufweisen. Diese Verbindungen werden als alkoxylierte Polyethylenimine bezeichnet und sind beispielsweise in US 5,846,453 und US 3,907,701 beschrieben. Auf die Angaben dieser Vorliteratur, insbesondere in Bezug auf die Herstellung der Polyamine, wird ausdrücklich Bezug genommen.

Die in den obigen Formeln (I) bis (III) gezeigten Strukturen sind idealisierte Formeln für den Fall, daß die dargestellten Polyethylenimine linear vorliegen. Die Wiederholungseinheiten können in beliebiger, beispielsweise statistischer, Abfolge vorliegen. Die beim erfindungsgemäßen Katalysatorsystem einzusetzenden Polyethylenimin-Polymere können auch teilweise verzweigt vorliegen und beispielsweise Strukturelemente der nachstehend gezeigten Art aufweisen:

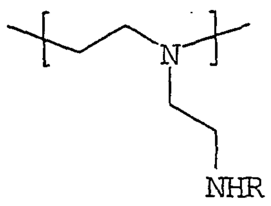

Wenn vorliegend im Zusammenhang mit Polyethyleniminen von "verzweigten Isomeren" die Rede ist, so sind damit Konstitutionsisomere angesprochen, die sich von der dargestellten Struktur durch ein- oder mehrfaches Einfügen einer der in den Formeln (I) bis (III) in Klammern dargestellten Wiederholungseinheiten zwischen eine NH-Bindung ableiten. Sie sind über tertiäre Stickstoffatome verzweigt.

Das Molekulargewicht der beim erfindungsgemäßen Katalysatorsystem zu verwendenden derivatisierten Polyamine beträgt mindestens 1000 g/mol vorzugsweise mehr als 10000 g/mol. Hierbei handelt es sich um eine mittlere Molmasse, da bei der Herstellung der Polyamine und weiterer Umsetzung wie üblich eine breite Molekulargewichtsverteilung eintritt.

Im Allgemeinen sind Polyethylenimine bevorzugt, die sulfonsäuregruppenfrei sind.

Polyethylenimine werden in der Regel durch Homo- oder Copolymerisation des Aziridins, gegebenenfalls mit anderen Monomeren, wie Vinylamiden, Vinylaminen, Acrylamiden, Acrylaminen, Acrylestern, Methacrylestern und Olefinen, wie Ethen, Propen, Buten oder Butadien, mit einem mittleren Molekulargewicht von 200 bis $2 \cdot 10^6$ g/mol hergestellt.

Die besonders bevorzugten acylierten Polyethylenimine (II) werden im Allgemeinen durch Umsetzung der Polyethylenimine mit Carbonsäuren, wie beispielsweise Ameisensäure, Essigsäure, Propionsäure, Buttersäure, Valeriansäure, Laurinsäure, 2-Ethylhexansäure, oder natürlichen $C_{18}$-Fettsäuren gewonnen, wobei der Amidierungsgrad 1 bis nahe 100%, vorzugsweise 30 bis nahe 100%, bezogen auf die amidierbaren Aminogruppen beträgt. Einzelheiten der Herstellung sind der Offenlegungsschrift DE-A 37 27 704 zu entnehmen. Es ist aber auch möglich, die acylierten Polyethylenimine (II) unter den Carbonylierungs-Reaktionsbedingungen in-situ darzustellen, beispielsweise durch Umsetzung eines Polyethylenimins mit einem Olefin und Kohlenmonoxid in Gegenwart der nukleophilen Verbindung, der Palladium-Komponente und des Phosphins oder durch Reaktion des Polyethylenimins mit der als Lösungsmittel verwendeten Carbonsäure unter den Bedingungen der Carbonylierungsreaktion.

Die besonders bevorzugten polyoxyalkylierten Polyethylenimine (III) werden im Allgemeinen durch Umsetzung der Polyethylenimine mit bis zu 500 mol Ethylenoxid, Propylenoxid oder Butylenoxid pro Monomereinheit des Polyethylenimins hergestellt. Einzelheiten der Herstellung sind der Patentschrift US 5,846,453 zu entnehmen.

Ganz besonders bevorzugt beim erfindungsgemäßen Katalysatorsystem sind die acylierten Polyethylenimine (I), welche die Strukturelemente (II) oder deren verzweigte Isomere aufweisen, in denen R' jeweils unabhängig voneinander $C_2$- bis $C_6$-Alkyl, wie beispielsweise Methyl, Ethyl, 1-Propyl, 2-Propyl (sek. Propyl), 1-Butyl, 2-Butyl (sek. Butyl), 2-Methyl-1-propyl (iso-Butyl), 2-Methyl-2-propyl (tert.-Butyl), 1-Pentyl, 2-Pentyl, 3-Pentyl, 3-Methyl-2-butyl und 2-Methyl-2-butyl bedeutet und die Summe aus m+n mindestens 50, insbesondere mindestens 100 beträgt.

Insbesondere umfasst das erfindungsgemäße Katalysatorsystem acylierte Polyethylenimine (II) oder deren verzweigte Isomere, in denen der Acylrest -CO-R' für Acylgruppen steht, welche dem herzustellenden Carbonsäurederivat entsprechen.

Das erfindungsgemäße Katalysatorsystem enthält im Allgemeinen 0,5 bis 15, bevorzugt 1 bis 10 und besonders bevorzugt 3 bis 7,5 Gew.-% Polyethylenimine, bezogen auf die Gesamtmasse des Reaktionsansatzes.

Für das erfindungsgemäße Katalysatorsystem geeignete Phosphine sind beispielsweise in den Offenlegungsschriften EP-A 0 274 795, EP-A 0 282 142, EP-A 0 386 833, EP-A 0 441 446, EP-A 0 495 547, EP-A 0 495 548, EP-A 0 499 329, EP-A 0 577 204, EP-A 0 577 205, WO 94/18154, WO 96/19434, WO 96/45040 und WO 98/42717 beschrieben, Die geeigneten Phosphine können durch die allgemeine Formel (VI)

$$PR^1R^2R^3 \qquad (VI),$$

in der die Reste $R^1$, $R^2$ und $R^3$ unabhängig voneinander für einen Kohlenstoff enthaltenden organischen Rest stehen, wiedergegeben werden. Die Reste $R^1$, $R^2$ und/oder $R^3$ können auch miteinander verbunden sein.

Unter einem Kohlenstoff enthaltenden organischen Rest ist ein unsubstituierter oder substituierter, aliphatischer, aromatischer oder araliphatischer Rest mit 1 bis 30 Kohlenstoffatomen zu verstehen. Dieser Rest kann ein oder mehrere Heteroatome, wie etwa Sauerstoff, Stickstoff, Schwefel oder Phosphor enthalten, beispielsweise -O-, -S-, -NR-, -CO-, -N=, $-SiR_2-$, -PR- und/oder $-PR_2$ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Enthält der Kohlenstoff enthaltende organische Rest ein oder mehrere Heteroatome, so kann dieser auch über ein Heteroatom gebunden sein. Somit sind beispielsweise auch Ether-, Thioether- und tertiäre Aminogruppen eingeschlossen. Bei dem Kohlenstoff enthaltenden organischen Rest kann es sich um einen einwertigen oder auch mehrwertigen, beispielsweise zweiwertigen Rest handeln.

Enthält das Phosphin (VI) genau ein Phosphoratom, d.h. die Reste $R^1$, $R^2$ und $R^3$ enthalten weder eine -PR- noch eine $-PR_2$-Gruppe, so wird dieses im Folgenden als monodentates Phosphin bezeichnet. Enthalten $R^1$, $R^2$ und/oder $R^3$ eine oder mehrere -PR- oder $-PR_2$-Gruppen, so werden die Phosphine (VI) entsprechend der Anzahl an Phosphoratomen als bidentat, tridentat, etc. bezeichnet.

Bevorzugt umfasst das erfindungsgemäße Katalysatorsystem ein mindestens bidentates Phosphin. Es kann durch die allgemeine Formel (VII)

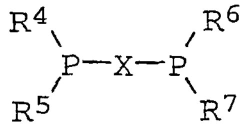

(VII),

in der die Reste $R^4$, $R^5$, $R^6$ und $R^7$ unabhängig voneinander einen Kohlenstoff enthaltenden organischen Rest bedeuten und X für eine Kohlenstoff enthaltende organische Brückengruppe steht, beschrieben werden. Die bevorzugten Phosphine (VII) sind bidentat, tridentat oder tetradentat, insbesondere bidentat.

Der Begriff Kohlenstoff enthaltender organischer Rest ist wie oben definiert.

Unter einer Kohlenstoff enthaltenden organischen Brückengruppe ist eine unsubstituierte oder substituierte, aliphatische, aromatische oder araliphatische zweiwertige Gruppe mit 1 bis 20 Kohlenstoffatomen und 1 bis 10 Atomen in der Kette zu verstehen. Die organische Brückengruppe kann ein oder mehrere Heteroatome, wie etwa Sauerstoff, Stickstoff, Schwefel oder Phosphor enthalten, beispielsweise -O-, -S-, -NR-, -CO-, -N=, $-SiR_2-$, -PR- und/oder $-PR_2$ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Enthält die organische Brückengruppe ein oder mehrere Heteroatome, so kann diese auch jeweils über ein Heteroatom gebunden sein. Somit sind beispielsweise auch Ether-, Thioether- und tertiäre Aminogruppen eingeschlossen.

Als einwertige Reste stehen $R^1$, $R^2$ und $R^3$ in Formel (VI) und $R^4$, $R^5$, $R^6$ und $R^7$ in Formel (VII) bevorzugt für

- einen unverzweigten oder verzweigten, acyclischen oder cyclischen, unsubstituierten oder substituierten Alkylrest mit 1 bis 20 aliphatischen Kohlenstoffatomen, bei dem eine oder mehrere der $CH_2$-Gruppen auch durch Heteroatome, wie -O- oder -S-, oder durch Heteroatom enthaltende Gruppen, wie -CO-,- NR- oder $-SiR_2-$, ersetzt sein können und bei dem ein oder mehrere der Wasserstoffatome durch Substituenten, wie beispielsweise Arylgruppen ersetzt sein können; oder für
- einen unsubstituierten oder substituierten aromatischen Rest mit einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl- oder Arylgruppen ersetzt sein können.

Als Beispiele der bevorzugten einwertigen Reste seien die unsubstituierten oder substituierten $C_1$- bis $C_{20}$-Alkyl-, $C_5$- bis $C_{20}$-Cycloalkyl-, $C_6$- bis $C_{20}$-Aryl- und $C_3$- bis $C_{20}$-Heteroaryl-Reste, wie beispielsweise Methyl, Ethyl, 1-Propyl, 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl, 3-Methyl-3-pentyl, 2-Methoxy-2-propyl, Cyclopentyl, Cyclohexyl, Cyclooctyl, Phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl, 2-Methoxyphenyl, 3-Methoxyphenyl, 4-Methoxyphenyl, 2-Pyridyl, 3-Pyridyl, 4-Pyridyl, 3-Pyridazinyl, 4-Pyridazinyl, 5-Pyridazinyl, 2-Pyrimidinyl, 4-Pyrimidinyl, 5-Pyrimidinyl, 2-Pyrazinyl, 2-(1,3,5-Triazin)yl, 1-Naphthyl, 2-Naphthyl, 2-Chinolyl, 8-Chinolyl, 1-Isochinolyl und 8-Isochinolyl genannt.

Als zweiwertige Reste stehen $R^1$ zusammen mit $R^2$, $R^2$ zusammen mit $R^3$ oder $R^1$ zusammen mit $R^3$ in Formel (VI) und $R^4$ zusammen mit $R^5$ und/oder $R^6$ zusammen mit $R^7$ in Formel (VII) bevorzugt für

- einen unverzweigten oder verzweigten, acyclischen oder cyclischen, unsubstituierten oder substituierten $C_4$- bis $C_{20}$-Alkylenrest ("zweiwertiger Alkylrest") mit 4 bis 10 Atomen in der Alkylenkette, bei dem $CH_2$-Gruppen auch durch Heterogruppen, wie beispielsweise -CO-, -O-, $-SiR_2-$ oder -NR- ersetzt sein können und bei dem ein oder mehrere

der Wasserstoffatome durch Substituenten, wie beispielsweise Arylgruppen ersetzt sein können.

Als Beispiele der bevorzugten zweiwertigen Reste seien die unsubstituierten oder substituierten $C_4$- bis $C_{30}$-Alkylen-Reste, bei denen $CH_2$-Gruppen durch Heterogruppen wie zum Beispiel durch -O- ersetzt sein können, wie beispielsweise 1,4-Butylen, 1,4-Dimethyl-1,4-butylen, 1,1,4,4-Tetramethyl-1,4-butylen, 1,4-Dimethoxy-1,4-butylen, 1,4-Dimethyl-1,4-dimethoxy-1,4-butylen, 1,5-Pentylen, 1,5-Dimethyl-1,5-pentylen, 1,5-Dimethoxy-1,5-pentylen, 1,2,5,5-Tetramethyl-2,5-pentylen, 1,5-Dimethyl-1,5-dimethoxy-1,5-pentylen, 3-Oxa-1,5-pentylen, 3-Oxa-1,5-dimethyl-1,5-pentylen, 3-Oxa-1,5-dimethoxy-1,5-pentylen, 3-Oxa-1,1,5,5-tetramethyl-1,5-pentylen, 3-Oxa-1,5-dimethyl-1,5-dimethoxy-1,5-pentylen, 1,4-Cyclooctylen, 1,5-Cyclooctylen, 1,4-Dimethyl-1,4-cyclooctylen, 1,4-Dimethyl-1,5-cyclooctylen, 1,4-Dimethyl-5,8-cyclooctylen, 1,5-Dimethyl-1,4-cyclooctylen, 1,5-Dimethyl-1,5-cyclooctylen, 1,5-Dimethyl-4,8-cyclooctylen,

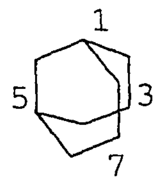

3,7-Bicyclo[3.3.1]nonylen,

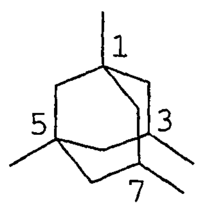

1,3,5,7-Tetramethyl-3,7-bicyclo[3.3.1]nonylen,

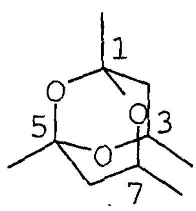

1,3,5,7-Tetramethyl-4,8,9-trioxa-3,7-bicyclo[3.3.1]nonylen,

genannt.

Als dreiwertige Reste stehen $R^1$ zusammen mit $R^2$ zusammen mit $R^3$ bevorzugt für

- einen unverzweigten oder verzweigten, acyclischen oder cyclischen, unsubstituierten oder substituierten dreiwertigen Alkyl-Rest mit 4 bis 20 Kohlenstoffatomen und jeweils 4 bis 10 Atomen in der Kette, bei dem $CH_2$-Gruppen auch durch Heterogruppen, wie beispielsweise -CO-, -O- oder -NR- ersetzt sein können und bei dem ein oder mehrere der Wasserstoffatome durch Substituenten, wie beispielsweise Arylgruppen ersetzt sein können.

Das beim erfindungsgemäßen Katalysatorsystem einzusetzende Phosphin (VI) beziehungsweise (VII) umfasst besonders bevorzugt

- Reste $R^1$, $R^2$ und/oder $R^3$ sowie $R^4$, $R^5$, $R^6$ und/oder $R^7$, welche unabhängig voneinander einen unsubstituierten oder substituierten $C_3$- bis $C_{12}$-Alkylrest, bei dem am $\alpha$-Kohlenstoffatom mindestens zwei, bevorzugt drei, weitere Gerüstatome gebunden sind, oder einen unsubstituierten oder substituierten aromatischen Rest mit 6 Ringatomen, bei dem ein, zwei oder drei Ringatome auch durch Stickstoff substituiert sein können; und/oder

- Reste $R^1$ zusammen mit $R^2$, $R^2$ zusammen mit $R^3$ oder $R^1$ zusammen mit $R^3$ sowie $R^4$ zusammen mit $R^5$ und/oder $R^6$ zusammen mit $R^7$, welche unabhängig voneinander einen unsubstituierten oder substituierten $C_4$- bis $C_{30}$-Alkylenrest mit 4 bis 7 Atomen in der kürzesten Alkylenkette, bei denen $CH_2$-Gruppen durch Heterogruppen wie zum Beispiel durch -O- ersetzt sein können;

bedeuten. Unter dem Begriff Gerüstatome sind die gerüstbildenden Atome, wie beispielsweise Kohlenstoff, Sauerstoff oder Stickstoff zu verstehen.

Als Beispiele der besonders bevorzugten einwertigen Reste $R^1$, $R^2$ und/oder $R^3$ sowie $R^4$, $R^5$, $R^6$ und/oder $R^7$ seien 2-Propyl (sek.-Propyl), 2-Butyl (sek.-Butyl), 2-Methyl-2-propyl (tert.-Butyl), 2-Methyl-2-butyl (tert.-Amyl), Phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl und 2-Pyridyl, insbesondere 2-Methyl-2-propyl (tert.-Butyl) und Phenyl, genannt. Als Beispiele der besonders bevorzugten zweiwertigen Reste $R^1$ zusammen mit $R^2$, $R^2$ zusammen mit $R^3$ oder $R^1$ zusammen mit $R^3$ sowie $R^4$ zusammen mit $R^5$ und/oder $R^6$ zusammen mit $R^7$ seien 1,1,4,4-Tetramethyl-1,4-butylen, 1,4-Dimethyl-1,4-dimethoxy-1,4-butylen, 1,1,5,5-Tetramethyl-1,5-pentylen, 1,5-Dimethyl-1,5-dimethoxy-1,5-pentylen, 1,5-Dimethyl-1,5-cyclooctylen, 1,3,5,7-Tetramethyl-3,7-bicyclo[3.3.1]nonylen und 1,3,5,7-Tetramethyl-4,8,9-trioxa-3,7-bicyclo[3.3.1]nonylen, insbesondere 1,3,5,7-Tetramethyl-4,8,9-trioxa-3,7-bicyclo[3.3.1]nonylen, genannt.

Die organische Brückengruppe X in Formel (VII) steht bevorzugt für eine unverzweigte oder verzweigte, acyclische oder cyclische, unsubstituierte oder substituierte zweiwertige aliphatische, aromatische oder araliphatische Gruppe mit 1 bis 20 Kohlenstoffatomen und 1 bis 8 Atomen, bevorzugt 2 bis 4 Atomen, in der Kette, bei der eine oder mehrere der $CH_2$-Gruppen auch durch Heteroatome, wie -O-, oder durch Heteroatom enthaltende Gruppen, wie -CO- oder -NR-, und/oder eines oder mehrere der aromatischen Ringatome durch Heteroatome, wie beispielsweise Stickstoff, substituiert sein können, und bei der ein oder mehrere der Wasserstoffatome durch Substituenten, wie beispielsweise Alkyl- oder Arylgruppen ersetzt sein können.

Als Beispiele der bevorzugten Brückengruppe X seien 1,2-Ethylen, 1,3-Propylen, 1,2-Propylen, 1,4-Butylen, 2-Methyl-1,3-propylen, 1,5-Pentylen, 2,2-Dimethyl-1,3-propylen, 1,6-Hexylen, $-O-CH_2CH_2-O-$, $-O-CH_2CH_2CH_2-O-$, $-CH_2-O-CH_2-$, $-CH_2CH_2-O-CH_2CH_2-$, o-Phenylen, o-Xylen und $-CH_2-NR-CH_2-$, insbesondere 1,2-Ethylen-, 1,3-Propylen-, 1,4-Butylen- und o-Xylen, genannt.

Als besonders bevorzugtes monodentates Phosphine sei Triphenylphosphin genannt. Als besonders bevorzugte bidentate Phosphine seien 1,2-Bis(di-tert.-butyl-phosphino)-ethan, 1,2-Bis(diphenylphosphino)-ethan, 1,2-P,P'-Di (2-phospha-1,3,5,7-tetramethyl-6,9,10-trioxatricyclo[3.3.1.1{3.7}]decyl-ethan (abgekürzt "dpa-2"), 1,3-Bis(di-tert.-butyl-phosphino)-propan, 1,3-Bis(diphenylphosphino)-propan, 1,3-P,P'-Di(2-phospha-1,3,5,7-tetramethyl-6,9,10-trioxatricyclo[3.3.1.1{3.7}]decyl-propan (abgekürzt "dpa-3"), 1,4-Bis(di-tert.-butyl-phosphino)-butan, 1,4-Bis(diphenylphosphino)-butan, 1, 4-P, P'-Di (2-phospha-1, 3, 5, 7-tetramethyl-6, 9,10-trioxatri-cyclo[3.3.1.1{3.7}]decyl-butan (abgekürzt "dpa-4"), α,α'-Bis(di-tert.-butyl-phosphino)-o-xylol, α,α'-Bis(diphenylphosphino)-o-xylol und α,α'-P,P'-Di(2-phospha-1,3,5,7-tetramethyl-6,9,10-trioxatricyclo[3.3.1.1{3.7}]decyl-o-xylol, insbesondere 1,3-Bis(di-tert. -butyl-phosphino) -propan, 1,3-Bis(diphenylphosphino)-propan und

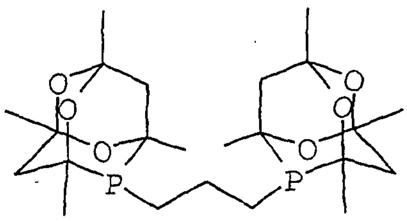

1,3-P,P'-Di(2-phospha-1,3,5,7-tetramethyl-6,9,10-trioxatricyclo[3.3.1.1{3.7}]decyl-propan

("dpa-3")

genannt. Das erfindungsgemäße Katalysatorsystem enthält beim Einsatz monodentater Phosphinliganden im Allgemeinen 50 bis 500, bevorzugt 100 bis 250 mmol Phosphin/l Reaktionsansatz und beim Einsatz bidentater Phosphinliganden im Allgemeinen 1 bis 50, bevorzugt 2 bis 25 mmol Phosphin/l Reaktionsansatz.

Als Palladium-Quelle kommen für das erfindungsgemäße Katalysatorsystem anorganische und organische Salze des Palladiums, Palladium-Verbindungen mit Stickstoff, Phosphor und/oder Sauerstoff-Donorliganden sowie auf einem Träger aufgebrachtes Palladium oder aufgebrachte Palladium-Verbindungen in Betracht. Bevorzugt sind halogenfreie Palladiumquellen.

Geeignete Beispiele anorganische und organische Salze des Palladiums sind Palladium(II)-nitrat, Palladium (II)-sulfat, Palladium(II)-carboxylate (zum Beispiel Palladium(II)-acetat oder Palladium(II)-propionat), Palladium(II)-sulfonate und Palladium(II)-acetylacetonat.

Geeignete Beispiele von Palladium-Verbindungen mit Stickstoff, Phosphor und/oder Sauerstoff-Donorliganden sind Tetrakis(triphenylphosphin)palladium(0) ($Pd(PPh_3)4$), Palladium(0)-dibenzylide-naceton ($Pd(dba)_2$) oder [Pd(dpa-3) $(CH_3CN)_2$] $[A]_2$, wobei A für ein schwach koordinierendes Anion, wie beispielsweise Chlorat, Hexafluorophosphat, Tetrafluoroborat oder p-Toluolsulfonat, steht und "dpa-3" wie oben definiert ist.

Als geeignetes Beispiel des auf einem Träger aufgebrachten Palladiums sei Palladium auf Aktivkohle genannt. Palladium-Quellen dieser Art werden bevorzugt als Partikel eingesetzt, welche eine Suspendierung im Reaktionsgemisch erlauben. Die Trägermaterialen sind zur Gewährleistung einer stabilen Suspension bevorzugt hydrophiler Natur, gegebenenfalls durch separate Maßnahmen, wie etwa oberflächliche Anoxidation von Aktivkohle, hydrophiliert.

Das erfindungsgemäße Katalysatorsystem enthält beim Einsatz monodentater Phosphinliganden im Allgemeinen 5 bis 20, bevorzugt 5 bis 10 mmol Palladium/l Reaktionsansatz und beim Einsatz bidentater Phosphinliganden im Allgemeinen 0,5 bis 5, bevorzugt 1 bis 3 mmol Palladium/l Reaktionsansatz.

Das erfindungsgemäße Katalysatorsystem enthält vorteilhafterweise als weitere Komponente eine Protonensäure (Brønsted-Säure) zur Aktivierung. Dabei kann es sich um die entsprechende Carbonsäure, welche bei der Carbonylierung der ungesättigten Verbindung mit Kohlenmonoxid und Wasser entsteht oder um eine andere organische oder anorganische Protonensäure handeln.

Geeignete Carbonsäuren, welche bei der Carbonylierung mit Kohlenmonoxid und Wasser gebildet werden können (in-situ) sind beispielsweise Propionsäure (aus Ethen), Buttersäure (aus Propen) oder Methacrylsäure (aus Propin). Die Carbonsäuren, z.B. Essigsäure oder Propionsäure, können natürlich auch zum Katalysatorsystem zugegeben werden (ex-situ). Wird das Katalysatorsystem zur Herstellung von Carbonsäuren eingesetzt, ist in Abhängigkeit von der Aktivität des Katalysatorsystems die in-situ gebildete Carbonsäure vielfach zur Aktivierung ausreichend. Der Vorteil dieser Variante ist, daß das System keine zusätzliche Komponente enthält.

In vielen Fällen ist es aber auch vorteilhaft, dem erfindungsgemäße Katalysatorsystem eine zusätzliche organische oder anorganische Protonensäure hinzuzufügen. Dies ist beispielsweise dann der Fall, wenn bei der Carbonylierung keine Carbonsäure zugegen ist oder in-situ gebildet wird (z.B. bei der Herstellung von Carbonsäureestern) oder ein, gegenüber der in-situ gebildeten Carbonsäure höherer aktivitätssteigernder Effekt erzielt werden soll. Beim Einsatz zusätzlicher Protonensäuren sind Protonensäuren mit einem $pK_a$-Wert von $\leq 3$, insbesondere von $\leq 2$, bevorzugt. Geeignete Protonensäuren sind beispielsweise in den Offenlegungsschriften EP-A 0 106 379 und EP-A 0 279 477 beschrieben, auf die hier ausdrücklich Bezug genommen wird. Bevorzugte Protonensäuren mit einem $pK_a$-Wert von $\leq 2$ sind beispielsweise die Phosphorsäure oder Sulfonsäuren, wie etwa p-Toluolsulfonsäure, Methylsulfonsäure oder Trifluormethylsulfonsäure. Werden Protonensäuren hinzugefügt, welche nicht dem herzustellenden Carbonsäurederivat entsprechen, so liegt deren Molverhältnis bezogen auf die Molmenge des eingesetzten Palladiums im Allgemeinen bei 1 bis 20 bezogen auf die Gesamtmasse des Reaktionsansatzes.

Das erfindungsgemäße Katalysatorsystem ist im Allgemeinen durch Zusammenfügen der oben.beschriebenen Komponenten (a), (b), (c) und gegebenenfalls einer Protonensäure in einem Lösungsmittel in beliebiger Reihenfolge und gegebenenfalls unter Bildung von zwischenstufen, wie etwa Palladium-Phosphin-Komplexen, erhältlich. Bevorzugt entspricht das Lösungsmittel dem gewünschten Carbonylierungsprodukt.

Des weiteren wurde ein Verfahren zur Carbonylierung von olefinisch oder acetylenisch ungesättigten Verbindungen mit Kohlenmonoxid und einer nukleophilen Verbindung gefunden, das dadurch gekennzeichnet ist, daß man das erfindungsgemäße Katalysatorsystem, welches vorangehend beschrieben ist, einsetzt.

Die einzelnen Komponenten des Katalysatorsystems und deren Mengenverhältnisse sind oben beschrieben.

Das erfindungsgemäße Verfahren führt man im Allgemeinen bei einer Temperatur von 50 bis 150, bevorzugt von 80 bis 150 und besonders bevorzugt von 90 bis 130°C durch. Bei Durchführung des erfindungsgemäßen Verfahrens beträgt der Druck im Allgemeinen 0,1 bis 5, bevorzugt 0,1 bis 3 und besonders bevorzugt 0,2 bis 1,5 MPa abs.

Als olefinisch ungesättigte Verbindungen können beim erfindungsgemäßen Verfahren unsubstituierte oder substituierte Alkene mit einer oder mehreren Kohlenstoff-Kohlenstoff-Doppelbindung/en eingesetzt werden. Als Substituenten kommen beispielsweise Aryl-, Heteroaryl-, Halogenide oder funktionelle Gruppen, wie etwa - COOH, -COOR, $-CONR_2$, -CN oder -OR in Betracht. Im Allgemeinen umfassen die olefinisch ungesättigte Verbindungen 2 bis 30 Kohlenstoffatome und eine oder zwei Kohlenstoff-Kohlenstoff-Doppelbindung/en. Als bevorzugte Verbindungen seien

- unsubstituierte $C_2$- bis $C_{20}$-Alkene, wie beispielsweise Ethen, Propen, 1-Buten, 2-Butene, 1-Penten, 1-Hexen, 3-Hexen, 1-Hepten, 3-Hepten, 1-Octen, 3-Methyl-2-hepten, 3-Methyl-3-hepten, 1-Decen, Cyclopenten, Cyclohexen oder Cycloocten;
- unsubstituierte $C_3$- bis $C_{20}$-Alkadiene mit kumulierten, konjugierten oder isolierten Kohlenstoff-Kohlenstoff-Doppelbindungen, wie beispielsweise Propadien (Allen), 1,3-Butadien, 1,3-Pentadien, 1,5-Hexadien oder Dicyclopentadien; und

- substituierte $C_3$- bis $C_{20}$-Alkene und Alkadiene, wie beispielsweise Styrol, 1-Methoxy-2-buten, 3-Methoxy-1-buten, 1-Butoxy-2-buten, 3-Butoxy-1-buten, Methyl-, Ethyl-, Propyl- oder Butyl-ester der 2-, 3- oder 4-Pentensäure oder 2-, 3- oder 4-Pentennitril;

genannt.

Als acetylenisch ungesättigte Verbindungen können beim erfindungsgemäßen Verfahren unsubstituierte oder substituierte Alkine mit einer oder mehreren Kohlenstoff-Kohlenstoff-Dreifachbindung/en eingesetzt werden. Im Allgemeinen umfassen sie 2 bis 30 Kohlenstoffatome und eine Kohlenstoff-Kohlenstoff-Dreifachbindung. Als Substituenten kommen die bei den olefinisch ungesättigten Verbindungen genannten in Betracht. Als bevorzugte Verbindungen seien

- unsubstituierte $C_2$- bis $C_{20}$-Alkine, wie beispielsweise Ethin oder Propin; und
- substituierte $C_3$- bis $C_{20}$-Alkine, wie beispielsweise Ethinylbenzol (Phenylacetylen) ;

genannt.

Als ungesättigte Verbindungen besonders bevorzugt eingesetzt werden die olefinisch ungesättigten Verbindungen, ganz besonders bevorzugt die unsubstituierten $C_2$- bis $C_6$-Alkene, insbesondere Ethen.

Das molare Verhältnis zwischen Kohlenmonoxid und den ungesättigten Verbindungen beträgt beim erfindungsgemäßen Verfahren im Allgemeinen 1 bis 3, bevorzugt 1 bis 1,5 pro Doppel- beziehungsweise Dreifachbindung.

Als nukleophile Verbindungen werden beim erfindungsgemäßen Verfahren Verbindungen eingesetzt, welche in der Regel mindestens ein mobiles Wasserstoffatom besitzen. Geeignete nukleophile Verbindungen sind beispielsweise Wasser, Alkohole und/oder Carbonsäuren. Bevorzugte Alkohole sind $C_1$- bis $C_{10}$-Alkanole, wie beispielsweise Methanol, Ethanol, 1-Propanol, 2-Propanol (Isopropanol), 1-Butanol, 2-Butanol (sek.-Butanol), 2-Methyl-1-propanol (Isobutanol), 2-Methyl-2-propanol (tert.-Butanol), 1-Pentanol, 2-Pentanol, 3-Pentanol, 2-Methyl-1-butanol, 3-Methyl-1-butanol (Isoamylalkohol), 2-Methyl-2-butanol (tert.-Amylalkohol), 1-Hexanol, 2-Hexanol, 4-Methyl-2-pentanol, 3,3-Dimethyl-2-butanol, 1-Heptanol, 1-Octanol, 1-Nonanol, 1-Decanol, 1,2-Ethylenglykol und 1,3-Propylenglykol. Bevorzugte Carbonsäuren sind $C_2$- bis $C_{20}$-Carbonsäuren, besonders bevorzugt $C_2$- bis $C_{10}$-Carbonsäuren, wie beispielsweise Essigsäure, Propionsäure, Buttersäure und Methacrylsäure, insbesondere jene Carbonsäuren, welche als Carbonylierungsprodukt der ungesättigten Verbindung mit Kohlenmonoxid und Wasser entstehen.

Beim Einsatz von Wasser als nukleophile Verbindung entstehen beim erfindungsgemäßen Verfahren Carbonsäuren, beim Einsatz von Alkoholen Carbonsäureester und beim Einsatz von Carbonsäuren (welche auch in-situ durch Einsatz von Wasser hergestellt worden sein können) Carbonsäureanhydride.

Besonders bevorzugt setzt man bei erfindungsgemäßen Verfahren als nukleophile Verbindung Wasser und/ oder ein $C_1$- bis $C_{10}$-Alkanol ein.

Das molare Verhältnis zwischen der ungesättigten Verbindung und der nukleophilen Verbindung kann beim erfindungsgemäßen Verfahren in einem sehr breiten Bereich variiert werden. Es beträgt im Allgemeinen 0,001 bis 1000 und bevorzugt 0,01 bis 1.

Als Carbonsäurederivate, welche nach dem erfindungsgemäßen Verfahren bevorzugt hergestellt werden, seien Propionsäure, Propionsäureanhydrid, Propionsäuremethylester, Propionsäurebutylester und Methacyrylsäuremethylester, insbesondere Propionsäure genannt .

Besonders bevorzugt setzt man beim erfindungsgemäßen Verfahren ein Polyethylenimine (I) ein, bei dem die Reste R Acylgruppen bedeuten, welche dem herzustellenden Carbonsäurederivat entsprechen. Bei der besonders bevorzugten Carbonylierung von Ethen wird somit vorzugsweise ein mit Propionsäure amidiertes Polyethylenimine (I) eingesetzt.

Das erfindungsgemäße Verfahren wird in überwiegend flüssiger Phase durchgeführt. Dabei ist das Katalysatorsystem im Allgemeinen weitesgehend homogen im Reaktionsgemisch solubilisiert. In der Regel fungieren die flüssigen Carbonylierungsprodukte, aber auch die nukleophilen Verbindungen und deren Gemische als Lösungsmittel. Es ist aber auch möglich, die Carbonylierung in einem bevorzugt inerten Lösungsmittel durchzuführen. Hierfür gut geeignet sind beispielsweise aromatische oder aliphatische Kohlenwasserstoffe, wie Toluol, Xylole oder Dekalin, oder polare, aprotische Lösungsmittel, wie Tetrahydrofuran, 1,4-Dioxan, N-Methyl-pyrrolidon, N-Methyl-piperidon, Dimethylsulfoxid, Glykolether (z.B. 1,2-Dimethoxyethan, Bis(2-methoxyethyl)-ether oder Bis(2-butoxyethyl)-ether), Dimethylformamid, Dimethylformanilid, Ethylencarbonat, Propylencarbonat, Ketone (z.B. Aceton oder Diethylketon) oder deren Gemische. Bevorzugt ist die Durchführung des Verfahrens in einem Lösungsmittel, was dem Carbonylierungsprodukt entspricht, da hierdurch keine weitere Fremdkomponente in das System eingebracht wird.

Das erfindungsgemäße Verfahren kann diskontinuierlich, halbkontinuierlich oder kontinuierlich durchgeführt werden.

In einer allgemeinen Ausführungsform wird das Katalysatorsystem beziehungsweise dessen Vorstufe durch Zufügen der Komponenten (a) Palladium-Quelle, (b) Phosphin, (c) im Reaktionsgemisch homogen gelöstes oder zumindest kolloidal verteiltes Polyethylenimin und gegebenenfalls einer Protonensäure in einem Lösungsmittel, in beliebiger Reihenfolge, hergestellt. Bevorzugt entspricht das Lösungsmittel dem gewünschten Carbonylierungsprodukt.

In einer allgemeinen Ausführungsform zur diskontinuierlichen Durchführung wird das Katalysatorsystem beziehungsweise dessen Vorstufe in einem geeigneten Reaktionsapparat (z.B. Autoklav) mit den Edukte ungesättigte Verbindung, nukleophile Verbindung und Kohlenmonoxid versetzt und das System unter Reaktionsbedingungen (Druck, Temperatur) belassen. Nach erfolgter Umsetzung wird abgekühlt, entspannt und der Reaktionsaustrag wie üblich aufgearbeitet, z.B. destillativ.

In einer allgemeinen Ausführungsform zur halbkontinuierlichen Durchführung wird das Katalysatorsystem beziehungsweise dessen Vorstufe in einem geeigneten Reaktionsapparat (z.B. Autoklav) je nach Ausführungsform mit keiner, mit einer, zwei oder allen drei Eduktkomponente/n versetzt und das System auf Reaktionsbedingungen (Temperatur, Druck) gebracht. Zur Einstellung des Druckes werden die gasförmigen Edukte Kohlenmonoxid und/oder die ungesättigte Verbindung (sofern gasförmig) zugeführt. Anschließend werden im Laufe der Reaktion die benötigten Edukte kontinuierlich oder periodisch zugeführt. Nach erfolgter Umsetzung wird abgekühlt, entspannt und der Reaktionsaustrag wie üblich aufgearbeitet, z.B. destillativ.

In einer allgemeinen Ausführungsform zur kontinuierlichen Durchführung wird das Katalysatorsystem beziehungsweise dessen Vorstufe in einem geeigneten Reaktionsapparat (z.B. Autoklav) je nach Ausführungsform mit keiner, mit einer, zwei oder allen drei Eduktkomponente/n versetzt und das System auf Reaktionsbedingungen (Temperatur, Druck) gebracht. Zur Einstellung des Druckes werden die gasförmigen Edukte Kohlenmonoxid und/oder die ungesättigte Verbindung (sofern gasförmig) zugeführt. Anschließend werden die drei Edukte entsprechend ihrem Verbrauch kontinuierlich zugeführt und ein entsprechender Teil des Reaktionsgemisches kontinuierlich aus dem Reaktionsapparat zur Aufarbeitung ausgeschleust.

In einer bevorzugten Ausführungsform zur kontinuierlichen Herstellung von Propionsäure werden Palladium (II)-acetat, der bidentate Phosphinligand "dpa-3" und ein mit Propionsäure amidiertes Polyethylenimin-Polymer in Propionsäure gelöst und in einem geeigneten Reaktionsapparat, beispielsweise einer Blasensäule, auf die gewünschte Reaktionstemperatur und durch Zufuhr von Kohlenmonoxid auf den gewünschten Reaktionsdruck gebracht. Anschließend beginnt man mit der kontinuierlichen Zufuhr der drei Edukte Ethen, Kohlenmonoxid und Wasser. Die gebildete Propionsäure kann nun beispielsweise durch Herausstrippen mit einem geeigneten Gasstrom (z.B. Ethen/Kohlenmonoxid-Gemisch) aus dem Reaktionsapparat kontinuierlich entfernt werden.

Das erfindungsgemäße Katalysatorsystem besitzt eine hohe katalytische Aktivität und bezüglich einer Abscheidung von Palladium und Palladiumverbindungen eine hohe Stabilität. Das erfindungsgemäße Verfahren unter Verwendung der erfindungsgemäßen Katalysatorsystems ermöglicht die Carbonylierung von olefinisch oder acetylenisch ungesättigten Verbindungen mit Kohlenmonoxid und einer nukleophilen Verbindung zu Carbonsäurederivaten unter milden Reaktionsbedingungen in hoher Ausbeute. Der Einsatz des stickstoffhaltigen Polymers ermöglicht zudem den entscheidenden Vorteil einer technisch einfachen Abtrennung des Carbonylierungsprodukts vom Reaktionsgemisch.

## Beispiele

Die in dieser Schrift verwendeten Größen sind, falls nicht anders erwähnt, wie untenstehend definiert, wobei PS für Propionsäure und PSA für Propionsäureanhydrid steht.

Selektivität (PS):

S(PS) = produzierte PS [mol] / {produzierte PS [mol] + produziertes Ethan [mol] + produziertes Diethylketon [mol] + produzierter Propionaldehyd [mol]}

Raum/Zeit-Ausbeute(PS) :

RZA(PS) = produzierte PS [g] / {Reaktionsvolumen [l] × Zeit [h]}

Raum/Zeit-Ausbeute(PSA):

RZA(PSA)= produzierter PSA [g] / {Reaktionsvolumen [l] × Zeit[h]}

Turn Over Frequency:

$$TOF = \{\text{produzierte PS [mol]} + \text{produziertes PSA [mol]}\} / \{\text{Palladium-Menge [mol]} \times \text{Zeit [h]}\}$$

Herstellung der Komponente A (amidiertes Polyethylenimin)

In einem Reaktionsgefäß wurden 500 g Polymin® WF (Bezugsquelle BASF Aktiengesellschaft) vorgelegt. Polymin® WF ist ein Polyethylenimin-Polymer mit einem gewichtsmittleren Molekulargewicht von ca. 25.000 g/mol. 500 g entsprechen etwa 11,6 mol Ethylenimin-Einheiten. Etwa 75% der vorhandenen Stickstoffatome können prinzipiell mittels Carbonsäuren amidiert werden, bei den restlichen 25% handelt es sich um tertiäre Amingruppen.

Bei einer Temperatur von 130°C wurden binnen 2 Stunden unter einem schwachen Stickstoffstrom 646 g (8,72 mol) Propionsäure hinzugetropft. Anschließend wurde die Temperatur auf 160°C erhöht und das Reaktionswasser abdestilliert, wobei etwa 34 g der zugesetzten Propionsäure (0,46 mol) mitgeschleppt wurden. Nach 4 Stunden wurde diese Menge an Propionsäure wieder hinzugefügt und das Reaktionsgemisch für 15 Stunden auf 180°C erhitzt. Nach dem Abkühlen wurden 964 g eines mit Propionsäure amidierten Polyethylenimins als gelb-roten Feststoff erhalten, was im Folgenden als "Polymin-PS" bezeichnet wird.

Herstellung der Komponente B (Diphosphinligand dpa-3)

Der Ausgangsstoff 1,3-Diphosphinopropan wurde nach R.W. Alder et al., J. Chem. Soc., Perkin Trans. I, 1998, Seite 1643 bis 1655 durch Umsetzung von 1,3-Dibrompropan mit Triethylphosphit zu 1,3-Bis(diethoxyphosphinyl)propan und anschließende Reduktion des isolierten Zwischenprodukts mit Lithiumaluminiumhydrid gewonnen.

Die Herstellung des Diphosphinligand 1,3-P,P'-Di(2-phospha-1,3,5,7-tetramethyl-6,9,10-trioxatricyclo[3.3.1.1{3.7}]decyl)propan, im Folgenden als "dpa-3" bezeichnet, erfolgte analog Beispiel 1 der WO 98/42717. Zu einer Lösung von 27,8 mmol 2,4-Pentandion in 20 mL 5M wässriger Chlorwasserstoffsäure wurden 4,63 mmol 1,3-Diphosphinopropan hinzugegeben und gerührt. Nach etwa einer Stunde begann die Ausfällung eines weißen Niederschlags. Nach weiteren 24 Stunden wurden die flüchtigen Bestandteile entfernt, das weiße Produkt mit Wasser (6-mal mit 20 mL) gewaschen, in 20 mL Dichlormethan gegeben und über Magnesiumsulfat getrocknet. Nach der Trocknung wurde das Trockenmittel abfiltriert und die Lösung im Vakuum auf etwa 1 mL eingeengt. Durch Zugabe von 10 mL wasserfreiem Pentan bildete sich erneut ein weißer Niederschlag an "dpa-3", welcher abgetrennt und von restlichem Lösungsmittel befreit wurde.

Versuchsdurchführung 1

Die Ausgangskomponenten des Katalysatorsystems (Palladium-Verbindung, Phosphin, gegebenenfalls ein Polyethylenimin und gegebenenfalls ein Aktivator) wurden unter Argonatmosphäre in entgaster wässriger Propionsäure gelöst und unter Argonatmosphäre in einen Autoklaven überführt. Der Autoklav wurde verschlossen und mit einem Kohlenmonoxid/Ethen-Gasgemisch mit einem molaren Verhältnis von 1:1 ein Druck von 0,6 MPa abs eingestellt. Anschließend wurde das Reaktionsgemisch auf die gewünschte Reaktionstemperatur aufgeheizt und durch Nachpressen mit dem Kohlenmonoxid/Ethen-Gasgemisch (molares Verhältnis 1:1) ein Druck von 1,1 MPa abs eingestellt. Der Druck wurde durch weiteres Nachpressen über die gesamte Versuchsdauer konstant gehalten. Nach einer Stunde wurde das Reaktionsgemisch auf Raumtemperatur abgekühlt, der Autoklav entspannt und das Reaktionsgemisch unter Argonatmosphäre ausgetragen. Die Zusammensetzung des Reaktionsgemisches und der Gasphase wurde gaschromatographisch beziehungsweise naßchemisch bestimmt.

Beispiel 1 (Stabilität des Katalysatorsystems)

Eine Lösung aus 0,87 g Tetrakis(triphenylphosphin)palladium ($Pd(PPh_3)_4$), 1,97 g Triphenylphosphin ($PPh_3$) und 3 g "Polymin-PS" (Komponente A) in 97 mL 98%-iger wässriger Propionsäure wurde unter Argonatmosphäre in einen Autoklaven überführt und 12 Tage bei 110°C unter 0,6 MPa abs Kohlenmonoxid-Atmosphäre gerührt. Zur Bestimmung des Palladium-Gehalts der Lösung wurden während des Versuchs Proben entnommen und mittels Atom-Absorptions-Spektroskopie (AAS) untersucht.

Die Ergebnisse sind graphisch in Abbildung 1 dargestellt. Während des Versuchs wurde keine Abscheidung von Palladium beobachtet. In der Lösung konnten nach Versuchsende nach 288 Stunden noch 98% des eingesetzten Palladiums nachgewiesen werden.

Beispiel 2 (Carbonylierung von Ethen, erfindungsgemäß)

55 g der thermisch mit Kohlenmonoxid vorbehandelten Reaktionslösung aus Beispiel 1 wurden mit 4 g Wasser und 1,5 g Triphenylphosphin ($PPh_3$) versetzt, mit Kohlenmonoxid/Ethen-Gasgemisch beschickt und wie in Versuchsdurchführung 1 beschrieben behandelt. Die Reaktionstemperatur betrug 110°C. Propionsäure wurde mit einer Raum/Zeit-Ausbeute von 87 g/1.h und einer Selektivität von über 99% gebildet. Die berechnete TOF betrug 170 $h^{-1}$.

Beispiel 3 (Carbonylierung von Ethen, erfindungsgemäß)

In einem Kontrollversuch mit einer frisch angesetzten, nicht mit Kohlenmonoxid vorbehandelten Reaktionslösung aus 0,17 g Palladiumacetat, 1,97 g Triphenylphosphin ($PPh_3$) und 3 g "Polymin-PS" (Komponente A) in 97 mL 90%-iger wässriger Propionsäure wurde nach Umsetzung mit einem Kohlenmonoxid/Ethen-Gasgemisch bei 110°C und Bedingungen wie unter Versuchsdurchführung 1 beschrieben Propionsäure mit einer Raum/Zeit-Ausbeute von 80 g/l·h und einer Selektivität von über 99% gebildet. Die berechnete TOF betrug 145 $h^{-1}$.

Ein Vergleich von Beispiel 2 mit 3 zeigt, daß das mit "Polymin-PS" stabilisierte Katalysatorsystem selbst nach einer 12-tägigen Einwirkung einer reduzierend wirkenden Kohlenmonoxid-Atmosphäre bei erhöhter Temperatur und einer weiteren Zugabe an Triphenylphosphin eine etwa vergleichbar hohe Aktivität und Selektivität aufweist wie ein frisch hergestelltes, nicht mit Kohlenmonoxid vorbehandeltes Katalysatorsystem.

Beispiel 4 (Stabilität des Katalysatorsystems)

Eine Lösung aus 0,34 g Palladiumacetat und 3,81 g Triphenylphosphin ($PPh_3$) in 200 mL 98%-iger wässriger Propionsäure wurde unter Argonatmosphäre in einen Autoklaven überführt und 8 Tage bei 110°C unter 0,6 MPa abs Kohlenmonoxid-Atmosphäre gerührt. Die Bestimmung des Palladium-Gehalts der Lösung erfolgte wie in Beispiel 1 über AAS.

Die Ergebnisse sind graphisch in Abbildung 1 dargestellt. Nach einer Versuchsdauer von 120 Stunden wurde die Abscheidung von elementarem Palladium beobachtet. In der Lösung konnten nach Versuchsende nach 192 Stunden nur noch etwa 60% des eingesetzten Palladiums nachgewiesen werden.

Beispiele 1 zeigt, daß in Gegenwart von "Polymin-PS" das Katalysatorsystem hervorragend stabilisiert ist, keine Abscheidung von elementarem Palladium stattfindet und der Palladiumgehalt in der Lösung auch nach mehreren Tagen nahezu vollständig erhalten bleibt. Im Gegensatz dazu wurde in Abwesenheit von "Polymin-PS" bereits nach 5 Tagen eine Abscheidung von elementarem Palladium unter signifikanter Abnahme des Palladiumgehalts in der Lösung beobachtet.

Beispiel 5 (Stabilität des Katalysatorsystems)

Die Ausgangsverbindung $[Pd(dpa\text{-}3)(CH_3CN)_2][OTs]_2$ des Katalysatorsystems wurde hergestellt durch sukzessive Umsetzung von Palladiumacetat mit "dpa-3" (Komponente B) und p-Toluolsulfonsäure (TsOH) in Acetonitril bei Raumtemperatur. Eine Lösung aus 0,8 g $[Pd(dpa\text{-}3)(CH_3CN)_2][OTs]_2$ (0,8 mmol) und 2,1 g "Polymin-PS" (Komponente A) in 68 mL 98%-iger wässriger Propionsäure wurde unter Argonatmosphäre in einen Autoklaven überführt und 5 Tage bei 110°C unter 0,6 MPa abs Kohlenmonoxid-Atmosphäre gerührt. Die Bestimmung des Palladium-Gehalts der Lösung erfolgte wie in Beispiel 1 über AAS.

Die Ergebnisse sind graphisch in Abbildung 2 dargestellt. Nach einer Versuchsdauer von 96 Stunden wurde die Abscheidung von elementarem Palladium beobachtet. In der Lösung konnten nach Versuchsende nach 120 Stunden noch etwa 75% des eingesetzten Palladiums nachgewiesen werden.

Beispiel 6 (Stabilität des Katalysatorsystems)

Beispiel 6 wurde ohne Zusatz von "Polymin-PS", ansonsten jedoch unter identischen Versuchsbedingungen wie Beispiel 5 durchgeführt.

Die Ergebnisse sind graphisch in Abbildung 2 dargestellt. Nach einer Versuchsdauer von 96 Stunden wurde die Abscheidung von elementarem Palladium beobachtet. In der Lösung konnten nach Versuchsende nach 120 Stunden nur noch etwa 40% des eingesetzten Palladiums nachgewiesen werden.

Beispiel 7 (Stabilität des Katalysatorsystems)

Beispiel 7 wurde mit einem weiteren Zusatz von 0,67 g "dpa-3" (1,4 mmol, Komponente B), ansonsten jedoch unter identischen Versuchsbedingungen wie Beispiel 5 durchgeführt.

Während des Versuchs wurde keine Abscheidung von Palladium beobachtet. In der Lösung konnten nach Versuchsende nach 120 Stunden noch über 95% des eingesetzten Palladiums nachgewiesen werden.

Ein Vergleich zwischen den Beispielen 5 und 6 zeigt, daß die Gegenwart von "Polymin-PS" die Stabilität des Katalysatorsystems deutlich erhöht und einer Abscheidung von elementarem Palladium entgegenwirkt. Eine Erhöhung des "dpa-3"/Palladium Verhältnisses von 1,0 in Beispiel 5 auf 2,75 in Beispiel 7 führt in Gegenwart von "Polymin-PS" zu einem sehr stabilen Katalysatorsystem, bei dem selbst nach 5 Tagen keine Abscheidung von elementarem Palladium beobachtet werden konnte und über 95% des eingesetzten Palladiums in der Lösung wiedergefunden werden konnte.

Beispiel 8 (Carbonylierung von Ethen, erfindungsgemäß)

0,17 g Palladiumacetat, 1,97 g Triphenylphosphin ($PPh_3$) und 3 g "Polymin-PS" (Komponente A) wurden unter Argonatmosphäre in 95 mL entgaster 98%-iger wässriger Propionsäure gelöst. Der Anteil an "Polymin-PS" betrug 3 Gew.-%, der Anteil an Wasser 2 Gew.-%, jeweils bezogen auf den gesamten flüssigen Reaktionsansatz. Anschließend wurde mit Kohlenmonoxid/Ethen-Gasgemisch beschickt und wie in Versuchsdurchführung 1 beschrieben behandelt. Die Reaktionstemperatur betrug 130°C.

Der Gehalt an Wasser im Austrag betrug 0,0 Gew.-%, bezogen auf den gesamten flüssigen Reaktionsaustrag. Die RZA(PS) betrug 58 g/l·h, die RZA(PSA) 160 g/l·h. Dies entspricht einer TOF von 260 $h^{-1}$.

Beispiel 9 (Carbonylierung von Ethen, Vergleichsbeispiel)

Beispiel 9 wurde ohne Zusatz von "Polymin-PS", ansonsten jedoch unter identischen Versuchsbedingungen wie Beispiel 8 durchgeführt.

Der Gehalt an Wasser im Austrag betrug 0,2 Gew.-%, bezogen auf den gesamten flüssigen Reaktionsaustrag. Die RZA(PS) betrug 42 g/l·h, die RZA(PSA) 90 g/l·h. Dies entspricht einer TOF von 170 $h^{-1}$.

Beispiel 8 zeigt, daß durch die Gegenwart von "Polymin-PS" eine signifikant höhere RZA an Propionsäure und Propionsäureanhydrid erreicht wird als in Beispiel 9 ohne Zusatz eines Polyethylenimins.

Beispiel 10 (Carbonylierung von Ethen, erfindungsgemäß)

0,17 g Palladiumacetat, 1,97 g Triphenylphosphin ($PPh_3$), 3 g "Polymin-PS" (Komponente A) und 2,2 g Phosphorsäure als Aktivator wurden unter Argonatmosphäre in 93 mL entgaster 97,5%-iger wässriger Propionsäure gelöst. Der Anteil an "Polymin-PS" betrug 3 Gew.-%, der Anteil an Wasser 2 Gew.-% und der Anteil an Phosphorsäure 2,3 Gew.-%, jeweils bezogen auf den gesamten flüssigen Reaktionsansatz. Anschließend wurde mit Kohlenmonoxid/Ethen-Gasgemisch beschickt und wie in Versuchsdurchführung 1 beschrieben behandelt. Die Reaktionstemperatur betrug 130°C.

Der Gehalt an Wasser im Austrag betrug 0,0 Gew.-%, bezogen auf den gesamten flüssigen Reaktionsaustrag. Die RZA(PSA) betrug 260 g/l·h. Da infolge der hohen Reaktionsgeschwindigkeit zum Propionsäureanhydrid etwa 7,8% der eingesetzten Propionsäure zusätzlich verbraucht wurden, wurde die RZA(PS) nicht berechnet. Die errechnete TOF betrug 410 $h^{-1}$.

Beispiel 11 (Carbonylierung von Ethen, Vergleichsbeispiel)

Beispiel 11 wurde ohne Zusatz von "Polymin-PS", ansonsten jedoch unter identischen Versuchsbedingungen wie Beispiel 10 durchgeführt.

Der Gehalt an Wasser im Austrag betrug 0,1 Gew.-%, bezogen auf den gesamten flüssigen Reaktionsaustrag. Die RZA(PS) betrug 98 g/l·h. Die errechnete TOF betrug 170 $h^{-1}$.

Beispiel 10 zeigt, daß durch die Gegenwart von "Polymin-PS" und einer starken Säure als Aktivator eine sehr hohe Reaktionsgeschwindigkeit erzielt wird, welche unter den eingestellten Reaktionsbedingungen zu einer Weiterreaktion der gebildeten und der bereits eingesetzten Propionsäure zum Propionsäureanhydrid führte. In Abwesenheit von "Polymin-PS" (Beispiel 11) wurde eine deutlich niedrigere Reaktionsgeschwindigkeit erreicht.

Die Beispiele 8 bis 11 zeigen, daß sowohl der Zusatz eines im Reaktionsgemisch homogen gelösten oder zumindest kolloidal verteilten Polyethylenimins als auch der Zusatz einer starken Säure als Aktivator einen entscheidenden Einfluß auf die Reaktionsgeschwindigkeit und die Ausbeute an Wertprodukten haben. Die mit Abstand besten Ergebnisse wurden in Gegenwart beider Zusätze (Polyethylenimin und starke Säure als Aktivator) erreicht.

Beispiel 12 (Carbonylierung von Ethen, Vergleichsbeispiel)

22 mg Palladiumacetat (0,1 mmol) und 57 mg "dpa-3" (0,12 mmol, Komponente B) wurden unter Argonatmosphäre in 100 mL entgaster 90%-iger wässriger Propionsäure gelöst. Anschließend wurde mit Kohlenmonoxid/ Ethen-Gasgemisch beschickt und wie in Versuchsdurchführung 1 beschrieben behandelt. Die Reaktionstemperatur betrug 100°C. Dabei entstand Propionsäure mit einer RZA(PS) von 110 g/l·h und einer Selektivität von über 99%. Die errechnete TOF betrug 1600 $h^{-1}$.

Beispiel 13 (Carbonylierung von Ethen, erfindungsgemäß)

22 mg Palladiumacetat (0,1 mmol), 142 mg "dpa-3" (0,3 mmol, Komponente B) und 7,5 g "Polymin-PS" (Komponente A) wurden unter Argonatmosphäre in 87,5 mL entgaster 85%-iger wässriger Propionsäure gelöst. Anschließend wurde mit Kohlenmonoxid/Ethen-Gasgemisch beschickt und wie in Versuchsdurchführung 1 beschrieben behandelt. Die Reaktionstemperatur betrug 100°C. Dabei entstand Propionsäure mit einer RZA(PS) von 345 g/1.h und einer Selektivität von über 99%. Die errechnete TOF betrug 4800 $h^{-1}$.

Beispiel 14 (Carbonylierung von Ethen, erfindungsgemäß)

Beispiel 14 wurde analog Beispiel 13 mit 22 mg Palladiumacetat (0,1 mmol), jedoch mit 57 mg "dpa-3" (0,12 mmol, Komponente B) und 3 g "Polymin-PS" (Komponente A) durchgeführt. Dabei entstand Propionsäure mit einer RZA (PS) von 340 g/l·h und einer Selektivität von über 99%. Die errechnete TOF betrug 4800 $h^{-1}$.

Die Beispiele 13 und 14 zeigen, daß in Gegenwart des bidentaten Phosphinliganden "dpa-3" und von "Polymin-PS" eine sehr hohe Raum/Zeit-Ausbeute an Propionsäure von deutlich über 300 g/l·h erhalten wird.

Beispiel 15 (Carbonylierung von Ethen, Vergleichsbeispiel)

2 g eines Suspensionskatalysators mit 5 Gew.-% Pd auf Aktivkohle und 57 mg "dpa-3" (0,12 mmol, Komponente B) wurden unter Argonatmosphäre in 100 mL entgaster 85%-iger wässriger Propionsäure gelöst. Anschließend wurde mit Kohlenmonoxid/Ethen-Gasgemisch beschickt und wie in Versuchsdurchführung 1 beschrieben behandelt. Die Reaktionstemperatur betrug 110°C. Dabei entstand Propionsäure mit einer RZA(PS) von nur 26 g/l·h und einer Selektivität von 95%.

Beispiel 16 (Carbonylierung von Ethen, erfindungsgemäß)

Beispiel 16 wurde analog Beispiel 15 mit 2 g eines Suspensionskatalysators mit 5 Gew.-% Pd auf Aktivkohle und 57 mg "dpa-3" (0,12 mmol, Komponente B), jedoch zusätzlich mit 5 g "Polymin-PS" (Komponente A) durchgeführt. Dabei entstand Propionsäure mit einer RZA(PS) von 460 g/l·h und einer Selektivität von über 99%.

Beispiel 16 zeigt, daß die eingesetzte Palladium-Komponente auch auf einem heterogenen Träger fixiert sein kann. Ein Vergleich mit Beispiel 15 macht dabei den deutlichen Effekt des im Reaktionsgemisch homogen gelösten oder zumindest kolloid verteilten Polyethylenimins deutlich. In Gegenwart von "Polymin-PS" konnte in Beispiel 16 gegenüber Beispiel 15 eine um Faktor 18 höhere Raum/Zeit-Ausbeute an Propionsäure erhalten werden.

## Patentansprüche

**1.** Katalysatorsystem zur Carbonylierung von olefinisch oder acetylenisch ungesättigten Verbindungen mit Kohlenmonoxid und einer nukleophilen Verbindung, enthaltend

(a) Palladium;
(b) ein Phosphin; und
(c) ein im Reaktionsgemisch homogen gelöstes oder zumindest kolloidal verteiltes, acyliertes Polyethylenimin, welches Einheiten der allgemeinen Formel (I) oder verzweigter Isomere davon

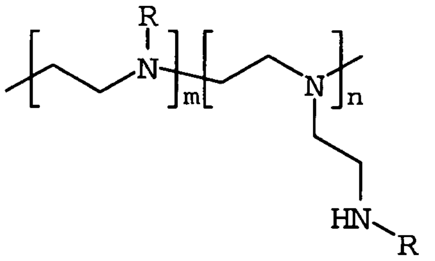

(I),

in der die Summe aus m+n mindestens 10 beträgt und R jeweils unabhängig voneinander Wasserstoff, Alkyl-, Cycloalkyl-, Aryl-, Aralkyl-, Acyl-Reste mit bis zu 30 Kohlenstoffatomen oder Hydroxyalkyl(poly)oxyalkylen-Reste mit bis zu 500 Alkylenoxy-Einheiten bedeuten, aufweist,
wobei das Polyethylenimin (I) die folgenden Strukturelemente (II) oder verzweigte Isomere davon

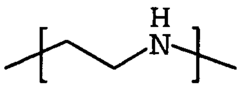

,

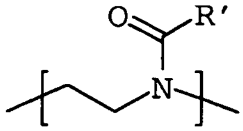

,

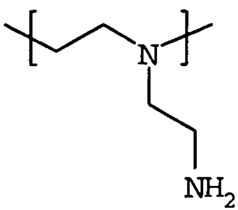

und

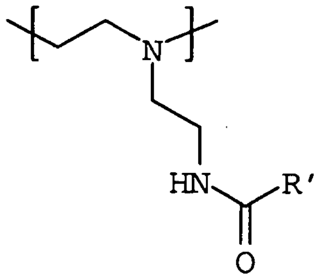

(II),

in denen R' unabhängig voneinander Alkyl mit 1 bis 29 Kohlenstoffatomen bedeutet, aufweist, und dessen Amidierungsgrad 1 bis nahe 100 %, bezogen auf die amidierbaren Aminogruppen beträgt.

2. Katalysatorsystem nach Anspruch 1, in dem das Polyethylenimin in einer Menge von 0,5 bis 15 Gew.-%, bezogen auf die Gesamtmasse des Reaktionsansatzes, vorhanden ist.

3. Katalysatorsystem nach den Ansprüchen 1 bis 2, enthaltend als Phosphin ein mindestens bidentates Phosphin.

4. Verfahren zur Carbonylierung von olefinisch oder acetylenisch ungesättigten Verbindungen mit Kohlenmonoxid und einer nukleophilen Verbindung, **dadurch gekennzeichnet, daß** man ein Katalysatorsystem gemäß den Ansprüchen 1 bis 3 einsetzt.

5. Verfahren nach Anspruch 4, **dadurch gekennzeichnet, daß** man die Carbonylierung bei einer Temperatur von 50 bis 150°C und einem Druck von 0,1 bis 5 MPa abs durchführt.

6. Verfahren nach den Ansprüchen 4 bis 5, **dadurch gekennzeichnet, daß** man als olefinisch ungesättigte Verbindung Ethen einsetzt.

7. Verfahren nach den Ansprüchen 4 bis 6, **dadurch gekennzeichnet, daß** man als nukleophile Verbindung Wasser, eine $C_2$- bis $C_{10}$-Carbonsäure und/oder ein $C_1$- bis $C_{10}$-Alkanol einsetzt.

8. Verfahren nach den Ansprüchen 4 bis 7, **dadurch gekennzeichnet, daß** man als Polyethylenimin ein Polyethylenimin (I), bei dem die Reste R Acylgruppen bedeuten, welche dem herzustellenden Carbonsäurederivat entsprechen, verwendet.

**Claims**

**1.** A catalyst system for the carbonylation of olefinically or acetylenically unsaturated compounds by means of carbon monoxide and a nucleophilic compound, comprising

(a) palladium,
(b) a phosphine, and
(c) an acylated polyethylenimine which is homogeneously dissolved or at least colloidally dispersed in the reaction mixture and comprises units of the formula (I) or branched isomers thereof.

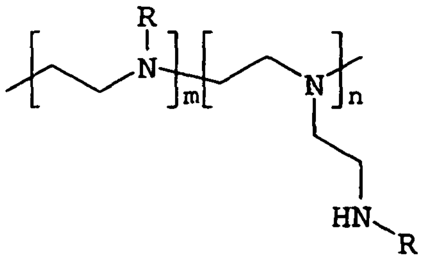

(I),

where the sum m+n is at least 10 and the radicals R are, independently of one another, hydrogen, alkyl, cycloalkyl, aryl, aralkyl and acyl radicals having up to 30 carbon atoms or hydroxyalkyl(poly)oxyalkylene radicals having up to 500 oxyalkylene units, wherein the polyethylenimine (I) comprises the following structural elements (II) or branched isomers thereof

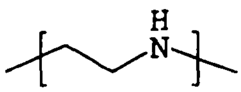 ,

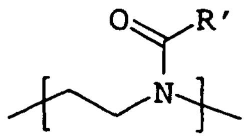 ,

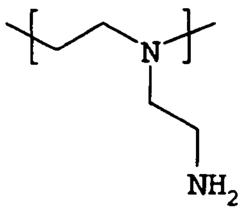

and

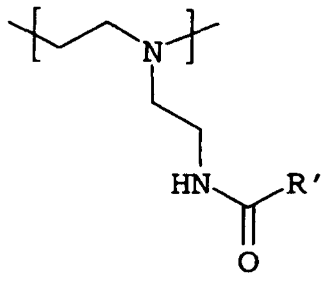

(II),

where the radicals R' are each, independently of one another, alkyl having from 1 to 29 carbon atoms, and has a degree of amidation of from 1 to close to 100%, based on the amidatable amino groups.

**2.** A catalyst system as claimed in claim 1 in which the polyethylenimine is present in an amount of from 0.5 to 15% by weight, based on the total mass of the reaction mixture.

**3.** A catalyst system as claimed in any of claims 1 to 12 in which the phosphine is an at least bidentate phosphine.

**4.** A process for the carbonylation of olefinically or acetylenically unsaturated compounds by means of carbon monoxide and a nucleophilic compound, wherein a catalyst system as claimed in any of claims 1 to 3 is used.

**5.** A process as claimed in claim 4, wherein the carbonylation is carried out at from 50 to 150°C and a pressure of from

0.1 to 5 MPa abs.

**6.** A process as claimed in claim 4 or 5, wherein the olefinically unsaturated compound used is ethene.

**7.** A process as claimed in any of claims 4 to 6, wherein the nucleophilic compound used is water, a $C_2$-$C_{10}$-carboxylic acid and/or a $C_1$-$C_{10}$-alkanol.

**8.** A process as claimed in any of claims 4 to 7, wherein the polyethylenimine used is a polyethylenimine (I) in which the radicals R are acyl groups which correspond to the carboxylic acid derivative to be prepared.

**Revendications**

**1.** Système catalytique pour la carbonylation de composés oléfiniquement ou acétyléniquement insaturés avec du monoxyde de carbone et un composé nucléophile, contenant

(a) du palladium,
(b) une phosphine, et
(c) une polyéthylèneimine acylée, qui est dissoute de manière homogène dans le mélange réactionnel ou au moins répartie de manière colloïdale et qui présente des unités de la formule générale (I) ou des isomères ramifiés de celle-ci

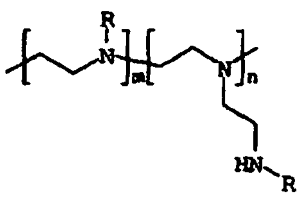 (I),

dans laquelle la somme de m+n est d'au moins 10 et R représente, chaque fois indépendamment, de l'hydrogène, des radicaux alkyle, cycloalkyle, aryle, aralkyle, acyle comportant jusqu'à 30 atomes de carbone ou des radicaux hydroxyalkyl-(poly)-oxyalkylène comportant jusqu'à 500 unités alkylèneoxy,
la polyéthylèneimine (I) présentant les éléments structurels suivants (II) ou des isomères ramifiés de ceux-ci

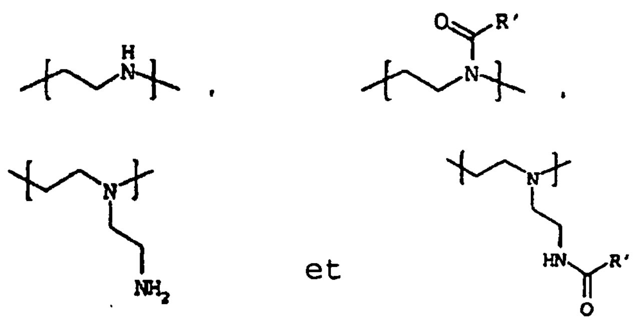
, , et (II),

où R' représente indépendamment un groupe alkyle comportant 1 à 29 atomes de carbone, et son degré d'amidation s'éléve de 1 jusqu'à proximité de 100 %, par rapport aux groupes amino amidables.

**2.** Système catalytique suivant la revendication 1, dans lequel la polyéthylèneimine est présente en une quantité de 0,5 à 15 % en poids, par rapport à la masse totale de la masse réactionnelle.

**3.** Système catalytique suivant les revendications 1 et 2,incontenant, comme phosphine, une phosphine au moins bidentate.

**4.** Procéde de carbonylation de composés oléfiniquement ou acétyléniquement insaturés avec du monoxyde de carbone et un composé nucléophile, **caractérisé en ce qu'**on met en oeuvre un système catalytique suivant les

revendications 1 à 3.

**5.** Procédé suivant la revendication 4, **caractérisé en ce qu'**on effectue la carbonylation à une température de 50 à 150°C et à une pression de 0,1 à 5 MPa abs.

**6.** Procédé suivant les revendications 4 et 5, **caractérisé en ce qu'**on met en oeuvre de l'éthène, comme composé oléfiniquement insaturé.

**7.** Procédé suivant les revendications 4 à 6, **caractérisé en ce que**, comme composé nucléophile, on met en oeuvre de l'eau, un acide carboxylique en $C_2$-$C_{10}$ et/ou un alcanol en $C_1$-$C_{10}$.

**8.** Procédé suivant les revendications 4 à 7, **caractérisé en ce que**, comme polyéthylèneimine, on utilise une polyéthylèneimine (I) dans laquelle les radicaux R représentent des groupes acyle qui correspondent au dérivé d'acide carboxylique à préparer.

FIG.1

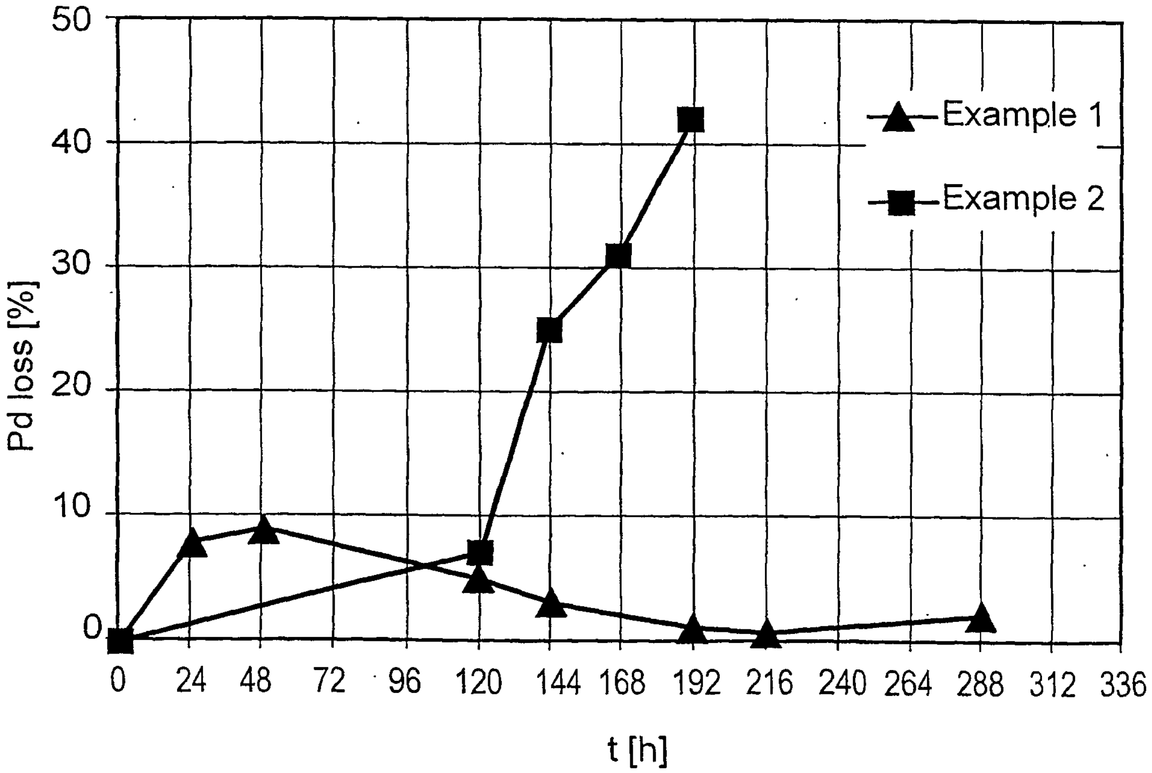
50
40
30
20
10
0
Pd loss [%]
0 24 48 72 96 120 144 168 192 216 240 264 288 312 336
t [h]
Example 1
Example 2

FIG.2

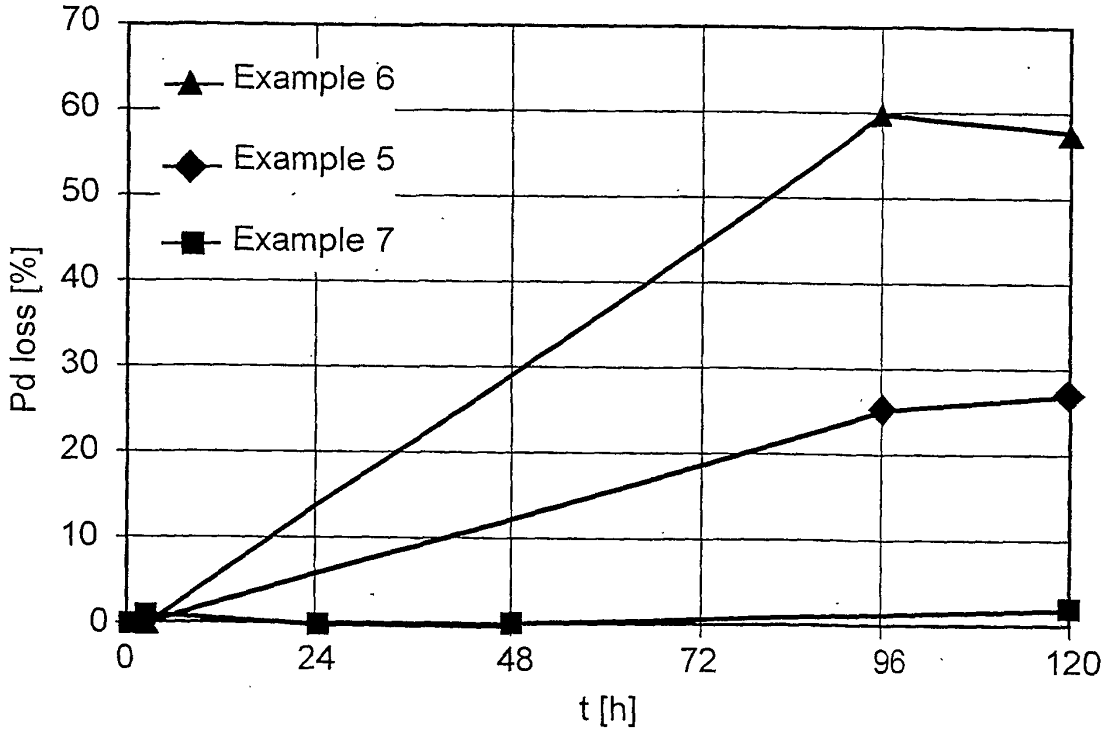
70
60
50
40
30
20
10
0
Pd loss [%]
Example 6
Example 5
Example 7
0
24
48
72
96
120
t [h]